# EUROPEAN PATENT APPLICATION

(11) **EP 1 229 111 A2**
(43) Date of publication of application: **07.08.2002**
(21) Application number: 02002650.6
(22) Date of filing: 05.02.2002
(51) Int. Cl.: C12N 5/04, A01H 4/00

(54) **Cultured cells of Ficus stipulata thunb. (= Ficus thunbergii) and a method for culturing tissues of the Ficus stipulata by using said cultured cells**

(30) Priority: 06.02.2001 JP 2001029640
(71) Applicant: Hiroshima University, Higashihiroshima City, Hiroshima Prefecture (JP)
(72) Inventor: Morikawa, Hiromichi, c/oHiroshima University, Higashihiroshima City, Hiroshima pref. (JP); Takahashi, Misa, c/oHiroshima University, Higashihiroshima City, Hiroshima pref. (JP)
(74) Representative: Henkel, Feiler, Hänzel

(57) **Abstract**

A cultured cell of *Ficus stipulata* Thunb. having high differentiating power, said cultured cell being obtained by culturing a part of a tissue of the *Ficus stipulata* Thunb. in a culture medium containing at least one kind of thidiazuron and benzyladenine in an amount effective to induce callus formation of the *Ficus stipulata* Thunb.

## Description

### Background of the Invention

### (1) Field of the Invention

The present invention relates to cultured cells and a tissue-culturing method, and particularly the invention relates to cultured cells of *Ficus stipulata* Thunb. (*=Ficus thunbergii*) and a method for culturing tissues of the *Ficus stipulata* Thunb. by using said cultured cells.

### (2) Related Art Statement

Recently, investigations have been vigorously carried out on the tissue culturing of the higher plants, and techniques for mass culturing plant tissues removed from leaves, stems, roots, etc. have public attracted attentions. When a plant piece cut off is place on agar or in a liquid containing a nutriment, a mass of cells swell up from a cut edge of the plant piece. This is called callus.

In general, cells of different tissues, for example, tissues of a root and a leaf, have different shapes and functions, respectively. This results from gradual differentiation during a time period in which the cells grow. However, the callus continue to grow without differentiation.

Generally speaking, seeding or cutting-planting must be done so as to propagate plants. Further, the soil and the environment largely influence the growth of the plants. However, the culturing of the callus is not influenced by changes in these matters. In addition, the callus grows faster than ordinary plant bodies. When a hormone or a chemical substance that promotes germination or rooting is added to the callus, a complete plant body is obtained.

As such a callus tissue-culturing method, methods for culturing tissues of trees such as poplar and eucalyptus are known.

However, what has been established as the tissue-culturing methods are limited to the kinds of trees such as poplar and eucalyptus only. One of reasons for this is that it is difficult to grow or root other breeds unless appropriate hormones or the like are used.

Recently, environmental pollution has been proceeding with NOx, SOx, volatile organic hydrocarbons, etc., which is feared to cause adverse effects upon plants, animals and human beings. Further, effects of the plants upon the heat island and the air pollution have been recognized, and wall-surface greening has been contrived in cities to green rooftops and walls faces of buildings. In particular, lian plants are suitable for greening cities in that they can grow, footing wall faces and rooftops of buildings. Therefore, effective propagation of such lian plants will be able to swiftly cope with greening the cities.

Among the lian plants, *Ficus stipulata* Thunb. is a plant belonging to the mordacious family, focus plants, and is a kind of roadside trees. *Ficus stipulata* Thunb. takes aerial roots in wall faces and grows along them. Since the leaves are as small as 1 to 2 cm in length, it is a lian plant suitable for greening the wall faces. In order to assuredly and swiftly provide such a lian plant, the tissue-culturing method is effective. However, a method for stably mass propagating the *Ficus stipulata* Thunb. has not been established yet up to now.

### Summary of the Invention

It is an object of the present invention to provide cultured cells and a tissue-cultivating method, which enable the establishment of stable mass propagation and transformation system of the *Ficus stipulata* Thunb.

In order to accomplish the above object, the present inventors had repeatedly made strenuous investigations on conditions suitable for inducing the formation of callus and the redifferentiation of the *Ficus stipulata* Thunb., and consequently have come to discover the cultured cells and the tissue-cultivating method according to the present invention.

The cultured cell having high differentiating power according to the present invention is obtained by culturing a part of a tissue of the *Ficus stipulata* Thunb. in a culture medium containing at least one kind of thidiazuron and benzyladenine in an amount effective to induce callus formation of the *Ficus stipulata* Thunb.

In a preferred embodiment of the cultured cell of the *Ficus* *stipulata* Thunb. according to the present invention, the tissue of the *Ficus stipulata* Thunb. is at least one kind of a tissue selected from the group consisting of a shoot apex, a stem, a leaf, an embryonic cell and a root.

In another preferred embodiment of the cultured cell of the *Ficus stipulata* Thunb. according to the present invention, the tissue of the *Ficus stipulata* Thunb. is a tissue originated from a plant that takes roots through its cutting of the *Ficus stipulata* Thunb. being aseptically planted thereto.

In a further preferred embodiment of the cultured cell of the *Ficus stipulata* Thunb. according to the present invention, the tissue of the *Ficus stipulata* Thunb. is a tissue less than 6 weeks after planting the cutting.

In a still further preferred embodiment of the cultured cell of the *Ficus stipulata* Thunb. according to the present invention, the culture medium is a WP culture medium or an MS culture medium.

The method for culturing a tissue of a *Ficus stipulata* Thunb. according to the present invention comprises the steps of subculturing any of the above cultured cells in a culture medium containing at least one kind of thidiazuron and benzyladenine in an amount effective to induce callus formation of the *Ficus stipulata* Thunb., and thereby obtaining a plantlet of the *Ficus stipulata* Thunb.

In a preferred embodiment of the *Ficus stipulata* Thunb. tissue-culturing method, the culture medium is a WP culture medium or an MS culture medium.

The cultured cell according to the present invention is a cultured cell of the *Ficus stipulata* Thunb. having high differentiating power, said cultured cell being obtained by culturing a part of a tissue of the *Ficus stipulata* Thunb. in a culture medium containing at least one kind of thidiazuron and benzyladenine in an amount effective to induce callus formation of the *Ficus stipulata* Thunb.

In general, the plantlet is formed according to the tissue-culturing method in the course of (1) formation of the callus, (2) formation of polyblast, (3) redifferentiation of the polyblast and (4) formation of the plantlet. Here, the term "polyblast" means a mass of numerous shoots in which cells are differentiated.

In the following, the cultured cells of *Ficus stipulata* Thunb. and the tissue-culturing method therefor according to the present invention will be explained.

Although the tissues of the *Ficus stipulata* Thunb. are not particularly limited, tissues from leaves, roots, stem apexes, root apexes, embryonic cells, etc. may be recited. Stem apexes, stems, leaves, embryonic cells and roots are preferred.

The tissues of the *Ficus stipulata* Thunb. may be ones taken from a matured tree of the *Ficus stipulata* Thunb. Preferably, the tissue is a tissue originated from a plant body that takes roots through the cutting the tissue of the *Ficus stipulata* Thunb. being planted. The aseptical treatment for the cutting is not particularly limited, for example, such a treatment can be effected by treating the tissue with ethanol or the like for a few or several hours and with sodium hypochlorite for a few or several hours and is washed with sterilized water.

As the cutting, an about 2 cm-long tip portion containing a shoot apex cut out can be used. The tissue of the *Ficus stipulata* Thunb. is preferably a tissue less than 6 weeks after planting the cutting.

The cultured cells of the present invention can be obtained by culturing a part of the tissue as mentioned above, in a culture medium containing at least one kind of thidiazuron and benzyladenine in an amount effective to induce callus formation of the *Ficus stipulata* Thunb. The amount of thidiazuron depends upon the culture medium used and the culturing condition, and is preferably 1 to 10⁴ nM. The reason for this range is to enhance the redifferentiation percentage. The amount of benzoadenine depends upon the culture medium used and the culturing condition, and is preferably 1 to 100 nM, more preferably 1 to 50 nM. The reason for this range is also to enhance the redifferentiation percentage.

As the culture medium, culture media such as a WP culture medium, an MS culture medium, a white culture medium and a modified culture medium may be recited. The WP culture medium is a cultured medium obtained by improving the MS culture medium for the purpose of the stem-tip culturing and mass culturing of ericaceous Kalmia latiflora. The MS culture medium is a culture medium developed aiming at the propagation of the marrow tissues and the callus of tobacco. In culturing the *Ficus stipulata* Thunb., the WP culture medium is preferred from the standpoint of enhancing the callus formation rate and the redifferentiation percentage.

In addition, the culture may contain trace amounts of organic substances, carbonaceous sources, etc. usually used in culturing. As the organic substances used in a trace amount, vitamins B1, B6, other vitamins such as nicotinic acid, thiamine hydrochloride, and pyridoxine hydrochloride, etc.; amino acids such as glycin, asparagine, etc.; hexavalent alcohols such as inosital, sorbit, etc. may be used.

As the carbonaceuous source, sugars such as sucrose, glycose, etc., may be recited.

The tissue piece planted in the culture medium can be cultured in a bright condition or a dark condition in a temperature range of 20 to 30°C. The temperature condition is preferably 22 to 28°C. Callus begins to be formed about one week after the culturing, and the callus is completely formed two weeks after the culturing. The stable callus is obtained by subculturing at a subculturing interval of about 10 to 20 days.

According to the tissue-culturing method of the present invention, a plantlet of the *Ficus stipulata* Thunb. can be obtained by subculturing the cultured cell having high differentiating power in a culture medium containing at least one kind of thidiazuron (TDZ) and benzyladenine (BA) in an amount effective to induce callus formation of the *Ficus stipulata* Thunb. As to the amounts of thidiazuron (TDZ) and benzyladenine (BA), the above-mentioned condition employed in culturing the cultured cells can be used.

As the cultured cells of the *Ficus stipulata* Thunb. having high differentiating power, the above-mentioned cultured cells according to the present invention can be used.

The growth of the plantlet can be promoted by cutting out polyblast and coating its cut edge with indoleacetic acid, for example.

As a support for the culture medium, pearlite, vermiculite, Gellan Gum, agar, agarose, etc. may be recited.

### (Experiments)

The present invention will be explained below in more detail based on experiments, but the invention is not to be interpreted as being limited to the following examples.

### Experiment 1

A culture medium in which the MS culture medium or the WP culture medium was used as a fundamental culture medium and 1% sucrose and 0.3 % Gellan Gum were added as the carbonaceuous source and a gelling agent, respectively, was used.

Cut pieces, 0.5 ∼ 1 mm long, were prepared from leaf, shoot and root tissues of the *Ficus stipulata* Thunb. asepatically cultured. The cut pieces of the leaf, shoot and root tissues of the *Ficus stipulata* Thunb. thus prepared were implanted on a culture medium containing thidiazuron and/or benzyladenine as plant hormones, thereby culturing the *Ficus stipulata* Thunb. One month after the culturing, it was observed that polyblasts were formed from the cut pieces. The polyblasts were cut out, their cut edge were coated with iondoleacetic acid, and the polyblasts were implanted into the above culture medium or a culture containing pearlite and vermiculite, and allowed to take roots. Table 1 shows rates of the cut pieces for which the formation of polyblast was observed and the number of shoots/cut piece.

**Table 1**

| BA (nM) | TDZ (nM) | Number of cut piece* | Number of shoots/ cut piece |
|---|---|---|---|
| 0 | 0 | 100 | 1.5 ± 1.0 |
| 0 | 1 | 100 | 1.5 ± 1.0 |
| 0 | 1 × 10² | 100 | 2.5 ± 0.6 |
| 0 | 1×10⁴ | 100 | 1.5 ± 1.0 |
| 9 | 0 | 50 | 1.8 ± 2.4 |
| 9 | 1 | 100 | 2.5 ± 0.6 |
| 9 | 1×10² | 100 | 2.5 ± 1.0 |
| 9 | 1×10⁴ | 75 | 1.5 ± 1.3 |
| 22 | 0 | 100 | 3.5 ± 0.6 |
| 22 | 1 | 100 | 3.0 ± 1.4 |
| 22 | 1×10² | 100 | 4.3 ± 1.9 |
| 22 | 1×10⁴ | 75 | 2.5 ± 1.7 |
| 36 | 0 | 100 | 2.5 ± 1.0 |
| 36 | 1 | 100 | 3.8 ± 1.3 |
| 36 | 1×10² | 100 | 3.5 ± 1.3 |
| 36 | 1×10⁴ | 100 | 2.3 ± 1.0 |
| 44 | 0 | 100 | 3.8 ± 0.5 |
| 44 | 1 | 100 | 4.0 ± 1.2 |
| 44 | 1×10² | 75 | 2.0 ± 1.4 |
| 44 | 1×10⁴ | 100 | 2.8 ± 1.3 |

| | | | |
|---|---|---|---|
| * (Number of cut pieces for which the formation of polyflasts was observed) × 100/cut piece | | | |

As is clear from Table 1, when cultured in the culture medium containing at least one of thidiazuron(TDZ) and/or benzyladenine (BA), the callus of the *Ficus stipulata* Thunb. could be induced at a high probability. Further, it was clarified that the redifferentiation of the *Ficus stipulata* Thunb. can be induced at a high probability by subculturing.

### Experiment 2

A redifferentiation condition most suitable for the introduction of a gene into the *Ficus stipulata* Thunb. was examined, and the gene was tried to be introduced thereinto.

First, the redifferentiation condition was examined. As the *Ficus stipulata* Thunb., one sterilized and aseptically subcultured was used. The *Ficus stipulata* Thunb. aseptically grown (4 to 6 weeks after implanting the cutting) used, and cut pieces were prepared from stem apexes, nodes and stem portions, and implanted in the culture medium. Six weeks later, the number and the shape of buds were observed. In culturing the *Ficus stipulata* Thunb., 1/3 of a block of Florialite (a mixture of vermiculite and cellulose fibers) was added into a test tube (30 x 200 mm), to which 15 ml of the MS culture medium or the WP culture medium was added. The test tube was closed with a polypropylene cap, which was placed in an autoclave.

A braird appearing in the *Ficus stipulata* Thunb. having been aseptically subcultured in an Agripot (a plastic container) was cut by about 2 to 3 cm, its cut section was immersed into a 1 g/l solution (1 g/l) of 4-(3-indolyl)butyric acid, and the cut piece was implanted in the test piece thus prepared, and subjected to 4 to 8 week culturing.

The culturing conditions were: atmospheric temperature 25°C, illumination intensity 30 ∼ 40µEm⁻²s⁻¹, a bright condition of 30 ∼ 40µEm⁻²s⁻¹ for 15 hours and a dark condition of 1 ∼ 2µEm⁻²s⁻¹ for 9 hours.

The MS culture medium or the WP culture medium was used as a fundamental culture medium. Sucrose 2% was added into each of the culture media, thereby adjusting the pH values of the MS culture medium and the WP culture medium to 5.6 and 5.2, respectively. Further, Gellan Gum 3% was added to the culture medium, and it was autoclaved at 120°C for 15 minutes, thereby preparing a solid culture medium. As plant hormones, naphthalanesulfonic acid (NAA), benzylaminopurine (BA), thidiazuron(TDZ), abscisic acid (ABA) and silver nitrate (AgNO₃) were used.

As a result, good redifferential state was exhibited in the use of BA and TDZ as the plant hormone, in the use of bright condition as the illumination state, and in the use of the WP culture medium as the culture medium.

Fig. 2 shows the number of cut pieces in which polyblasts were formed at step apex and the number of buds formed.

**Table 2**

| BA (nM) | TDZ (nM) | Total number of cut pieces | Number of cut pieces* | Number of buds formed |
|---|---|---|---|---|
| 0 | 0 | 4 | 4 | 1.5 ± 1.0 |
| 0 | 1 | 4 | 4 | 1.5 ± 1.0 |
| 0 | 10² | 4 | 4 | 2.5 ± 0.6 |
| 0 | 10⁴ | 4 | 4 | 1.5 ± 1.0 |
| 8.9 | 0 | 4 | 2 | 1.8 ± 2.4 |
| 8.9 | 1 | 4 | 4 | 2.5 ± 0.6 |
| 8.9 | 10² | 4 | 4 | 2.5 ± 1.0 |
| 8.9 | 10⁴ | 4 | 3 | 1.5 ± 1.3 |
| 22.2 | 0 | 4 | 4 | 3.5 ± 0.6 |
| 22.2 | 1 | 4 | 4 | 3.0 ± 1.4 |
| 22.2 | 10² | 4 | 4 | 4.3 ± 1.9 |
| 22.2 | 10⁴ | 4 | 3 | 2.5 ± 1.7 |
| 35.5 | 0 | 4 | 4 | 2.5 ± 1.0 |
| 35.5 | 1 | 4 | 4 | 3.8 ± 1.3 |
| 35.5 | 10² | 4 | 4 | 3.5 ± 1.3 |
| 35.5 | 10⁴ | 4 | 4 | 2.3 ± 1.0 |
| 44.4 | 0 | 4 | 4 | 3.8 ± 0.5 |
| 44.4 | 1 | 4 | 4 | 4.0 ± 1.2 |
| 44.4 | 10² | 4 | 3 | 2.0 ± 1.4 |
| 44.4 | 10⁴ | 4 | 4 | 2.8 ± 1.3 |

| | | | | |
|---|---|---|---|---|
| * Number of cut pieces in which polyflasts were formed at a stem apex. | | | | |

### Experiment 3

Next, transformed bodies of the *Ficus stipulata* Thunb. were tried to be prepared. A gene was introduced by a particle gun method. First, an expression vector pTH-2 in which a GFP (Green Fluorescence Protein) gene was introduced into a high efficiency gene expression cassette of a plant. As the plant, an explant cultured for 3 ∼ 14 days in the above-mentioned culturing condition for the formation of the polyblasts was used. A number of GFP signals were observed 2 days after the introduction of the gene. This evidenced that the GFP gene was accurately introduced into the *Ficus stipulata* Thunb.

The cultured cells according to the present invention exhibits the advantageous effect that they can used for the transformation of the *Ficus stipulata* Thunb.

Further, the tissue-culturing method according to the present invention exhibits the advantageous effect that the *Ficus stipulata* Thunb. can be mass propagated and can thus be used as a material for greening the wall faces.

## Claims

1. A cultured cell of *Ficus stipulata* Thunb. having high differentiating power, said cultured cell being obtained by culturing a part of a tissue of the *Ficus stipulata* Thunb. in a culture medium containing at least one kind of thidiazuron and benzyladenine in an amount effective to induce callus formation of the *Ficus stipulata* Thunb.

2. The cultured cell set forth in claim 1, wherein the tissue of the *Ficus stipulata* Thunb. is at least one kind of a tissue selected from the group consisting of a shoot apex, a stem, a leaf, an embryonic cell and a root.

3. The cultured cell set forth in claim 1 or 2, wherein the tissue of the *Ficus stipulata* Thunb. is a tissue originated from a plant body that takes roots through a cutting of the *Ficus stipulata* Thunb. being implanted therein.

4. The cultured cell set forth in claim 3, wherein the tissue of the *Ficus stipulata* Thunb. is a tissue less than 6 weeks after implanting the cutting.

5. The cultured cell set forth in any one of claims 1 to 4 wherein the culture medium is a WP culture medium or an MS culture medium.

6. A method for culturing a tissue of a *Ficus stipulata* Thunb., comprising the steps of subculturing the cultured cell set forth in any one of claims 1 to 5 in a culture medium containing at least one kind of thidiazuron and benzyladenine in an amount effective to induce callus formation of the *Ficus stipulata* Thunb., and thereby obtaining a plantlet of the *Ficus stipulata* Thunb.

7. The method set forth in claim 6, wherein the culture medium is a WP culture medium or an MS culture medium.
